# EUROPEAN PATENT APPLICATION

(11) **EP 1 106 210 A2**
(43) Date of publication of application: **13.06.2001**
(21) Application number: 00310719.0
(22) Date of filing: 01.12.2000
(51) Int. Cl.: A61P 3/10, A61K 45/06

(54) **Combination of aldose reductase inhibitors and antihypertensive agents for the treatment of diabetic complications**

(30) Priority: 07.12.1999 US 169380 P
(71) Applicant: Pfizer Products Inc., Groton, Connecticut 06340 (US)
(72) Inventor: Mylari, Banavara Lakshman, Groton, Connecticut 06340 (US)
(74) Representative: Sewell, Richard Charles

(57) **Abstract**

This invention is directed to methods, pharmaceutical compositions and kits comprising an aldose reductase inhibitor (ARI), a prodrug thereof or a pharmaceutically acceptablre salt of said ARI or said prodrug and an antihypertensive agent, a prodrug thereof or a pharmaceutically acceptable salt of said antihypertensive agent or said prodrug. This invention further relates to methods of using those pharmaceutical compositions for the treatment of diabetic complications such as diabetic neuropathy, diabetic nephropathy, diabetic retinopathy, myocardial infarction, cataracts and diabetic cardiomyopathy.

## Description

### BACKGROUND OF THE INVENTION

This invention relates to methods, pharmaceutical compositions and kits comprising an aldose reductase inhibitor (ARI), a prodrug thereof or a pharmaceutically acceptable salt of said ARI or said prodrug and an antihypertensive agent, a prodrug thereof or a pharmaceutically acceptable salt of said antihypertensive agent or said prodrug. This invention further relates to methods of using such pharmaceutical compositions for the treatment of diabetic complications such as diabetic neuropathy, diabetic nephropathy, diabetic retinopathy, myocardial infarction, cataracts and diabetic cardiomyopathy.

Aldose reductase inhibitors function by inhibiting the activity of the enzyme aldose reductase, which is primarily responsible for regulating the reduction of aldoses, such as glucose and galactose, to the corresponding polyols, such as sorbitol and galactitol, in humans and other animals. In this way, unwanted accumulations of galactitol in the lens of galactosemic subjects and of sorbitol in the lens, peripheral nervous cord and kidneys of various diabetic subjects are prevented or reduced. Accordingly, aldose reductase inhibitors are of therapeutic value for controlling certain diabetic complications, e.g., diabetic neuropathy, diabetic nephropathy, diabetic cardiomyopathy, myocardial infarction, cataracts and diabetic retinopathy.

Several classes of compounds are known to have activity as antihypertensive agents. These include calcium channel blockers, A-II antagonists, diuretics, beta- . adrenergic receptor blockers, neutral endopeptidase inhibitors, vasodilators and alpha-adrenergic receptor blockers. International Patent Application Publication Number WO99/02189, published January 21, 1999, discloses the use of ACE inhibitors in combination with aldose reductase inhibitors to treat diabetic complications such as diabetic neuropathy, diabetic nephropathy and diabetic retinopathy.

### SUMMARY OF THE INVENTION

This invention is directed to pharmaceutical compositions comprising an aldose reductase inhibitor (ARI), a prodrug thereof or a pharmaceutically acceptable salt of said ARI or of said prodrug; an antihypertensive agent, a prodrug thereof or a pharmaceutically acceptable salt of said antihypertensive agent or of said prodrug; and a pharmaceutically acceptable carrier, vehicle or diluent.

This invention is also directed to methods of treating a diabetic complication in a mammal comprising administering to said mammal a pharmaceutical composition as set forth hereinbelow. In particular, such diabetic complications as, for example, diabetic neuropathy, diabetic nephropathy, diabetic cardiomyopathy, myocardial infarction, cataracts and diabetic retinopathy can be treated by the methods of this invention.

This invention is also directed to methods of treating a diabetic complication in a mammal comprising administering to said mammal an ARI, a prodrug thereof or a pharmaceutically acceptable salt of said ARI or said prodrug; and an antihypertensive agent, a prodrug thereof or a pharmaceutically acceptable salt of said antihypertensive agent or said prodrug.

This invention is especially directed to methods wherein the ARI, prodrug thereof or pharmaceutically acceptable salt of said ARI or said prodrug, and the antihypertensive agent, prodrug thereof or pharmaceutically acceptable salt of said antihypertensive agent, are administered separately.

This invention is also especially directed to methods wherein the ARI, prodrug thereof or pharmaceutically acceptable salt of said ARI or said prodrug, and the antihypertensive agent, prodrug thereof or pharmaceutically acceptable salt of said antihypertensive agent or said prodrug are administered together.

This invention is also directed to kits comprising:
a) a first unit dosage form comprising an aldose reductase inhibitor (ARI), a prodrug thereof or a pharmaceutically acceptable salt of said ARI or said prodrug and a pharmaceutically acceptable carrier, vehicle or diluent;
b) a second unit dosage form comprising an antihypertensive agent, a prodrug thereof or a pharmaceutically acceptable salt of said antihypertensive agent or said prodrug and a pharmaceutically acceptable carrier, vehicle or diluent; and
c) a container.

In the compositions, methods and kits of this invention, it is preferred that said ARI is fidarestat, epalrestat, minalrestat, SPR-210, zenarastat or zopolrestat, a prodrug thereof or a pharmaceutically acceptable salt of said ARI or of said prodrug. It is especially preferred that said ARI is zopolrestat, a prodrug thereof or a pharmaceutically acceptable salt thereof or of said prodrug.

In the compositions, methods and kits of this invention, it is preferred that said antihypertensive agent is a compound selected from the following classes of antihypertensive agents: calcium channel blockers, A-II antagonists, diuretics, endopeptidase inhibitors, beta-adrenergic receptor blockers and alpha-adrenergic receptor blockers.

Preferred calcium channel blockers include verapamil, diltiazem, mibefradil, isradipine, lacidipine, nicardipine, nifedipine, amlodipine, nimodipine, nisoldipine, nitrendipine and felodipine, prodrugs thereof and pharmaceutically acceptable salts of said calcium channel blockers and said prodrugs. Especially preferred calcium channel blockers include nifedipine, amlodipine and felodipine, prodrugs thereof and pharmaceutically acceptable salts of said calcium channel blockers and said prodrugs.

Preferred A-II antagonists include losartan, irbesartan and valsartan, prodrugs thereof and pharmaceutically acceptable salts of said A-II antagonists and said prodrugs.

Preferred diuretics include amiloride and bendroflumethiazide, prodrugs thereof and pharmaceutically acceptable salts of said diuretics and said prodrugs.

Endopeptidase inhibitors may be neutral endopeptidase inhibitors or may be dual ACE inhibitor/neutral endopeptidase inhibitors. Preferred neutral endopeptidase inhibitors include candoxatril and candoxatrilat, prodrugs thereof and pharmaceutically acceptable salts thereof and of said prodrugs. Preferred dual ACE inhibitor/neutral endopeptidase inhibitors include sampatrilat and omapatrilat, prodrugs thereof and pharmaceutically acceptable salts thereof and of said prodrugs. Sampatrilat and omapatrilat are dual ACE inhibitor/neutral endopeptidase inhibitors. Omapatrilat is also known as a vasopeptidase inhibitor.

A preferred beta-adrenergic receptor blocker is carvedilol, prodrugs thereof and pharmaceutically acceptable salts of said beta-adrenergic receptor blockers and said prodrugs.

Preferred alpha-adrenergic receptor blockers include doxazosin, prazosin and trimazosin, prodrugs thereof and pharmaceutically acceptable salts of said alpha-adrenergic receptor blockers and of said prodrugs.

### DETAILED DESCRIPTION OF THE INVENTION

The methods, compositions and kits of this invention are useful in treating diabetic complications, including, but not limited to, diabetic neuropathy, diabetic nephropathy, diabetic cardiomyopathy, myocardial infarction, cataracts and diabetic retinopathy.

The term "treating", as used herein, refers to retarding, arresting or reversing the progress of, or alleviating or preventing either the disorder or condition to which the term "treating" applies, or one or more symptoms of such disorder or condition. The term "treatment", as used herein, refers to the act of treating a disorder, symptom or condition, as the term "treating" is defined above.

Any aldose reductase inhibitor may be used in the pharmaceutical compositions, methods and kits of this invention. The term aldose reductase inhibitor refers to a compound which inhibits the bioconversion of glucose to sorbitol catalyzed by the enzyme aldose reductase. Such inhibition is readily determined by those skilled in the art according to standard assays (J. Malone, Diabetes, 29:861-864, 1980. "Red Cell Sorbitol, an Indicator of Diabetic Control"). The following patents and patent applications, each of which is hereby wholly incorporated herein by reference, exemplify aldose reductase inhibitors which can be used in the compositions, methods and kits of this invention, and refer to methods of preparing those aldose reductase inhibitors: United States Patent 4,251,528; United States Patent 4,600,724; United States Patent 4,464,382, United States Patent 4,791,126, United States Patent 4,831,045; United States Patent 4,734,419; 4,883,800; United States Patent 4,883,410; United States Patent 4,883,410; United States Patent 4,771,050; U.S. 5,252,572; United States Patent 5,270,342; U.S. 5,430,060; United States Patent 4,130,714; United States Patent 4,540,704; United States Patent 4,438,272; United States Patent 4,436,745, United States Patent 4,438,272; United States Patent 4,436,745, United States Patent 4,438,272; United States Patent 4,436,745, United States Patent 4,438,272; United States Patent 4,980,357; United States Patent 5,066,659; United States Patent 5,447,946; United States Patent 5,037,831.

A variety of aldose reductase inhibitors are specifically described and referenced below, however, other aldose reductase inhibitors will be known to those skilled in the art. Also, common chemical USAN names or other designations are in parentheses where applicable, together with reference to appropriate patent literature disclosing the compound.

Accordingly, examples of aldose reductase inhibitors useful in the compositions, methods and kits of this invention include:
1. 3-(4-bromo-2-fluorobenzyl)-3,4-dihydro-4-oxo-1-phthalazineacetic acid (ponalrestat, US 4,251,528);
2. N[[(5-trifluoromethyl)-6-methoxy-1-naphthalenyl]thioxomethyl)-N-methylglycine (tolrestat, US 4,600,724);
3. 5-[(Z,E)-β-methylcinnamylidene]-4-oxo-2-thioxo-3-thiazolideneacetic acid (epalrestat, US 4,464,382, US 4,791,126, US 4,831,045);
4. 3-(4-bromo-2-fluorobenzyl)-7-chloro-3,4-dihydro-2,4-dioxo-1(2H)-quinazolineacetic acid (zenarestat, US 4,734,419, and US 4,883,800);
5. 2R,4R-6,7-dichloro-4-hydroxy-2-methylchroman-4-acetic acid (US 4,883,410);
6. 2R,4R-6,7-dichloro-6-fluoro-4-hydroxy-2-methylchroman-4-acetic acid (US 4,883,410);
7. 3,4-dihydro-2,8-diisopropyl-3-oxo-2H-1,4-benzoxazine-4-acetic acid (US 4,771,050);
8. 3,4-dihydro-3-oxo-4-[(4,5,7-trifluoro-2-benzothiazolyl)methyl]-2H-1,4-benzothiazine-2-acetic acid (SPR-210, U.S. 5,252,572);
9. N-[3,5-dimethyl-4-[(nitromethyl)sulfonyl]phenyl)-2-methyl-benzeneacetamide (ZD5522, U.S. 5,270,342 and U.S. 5,430,060);
10. (S)-6-fluorospiro[chroman-4,4'-imidazolidine]-2,5'-dione (sorbinil, US 4,130,714);
11. d-2-methyl-6-fluoro-spiro(chroman-4',4'-imidazolidine)-2',5'-dione (US 4,540,704);
12. 2-fluoro-spiro(9H-fluorene-9,4'-imidazolidine)-2',5'-dione (US 4,438,272);
13. 2,7-di-fluoro-spiro(9H-fluorene-9,4'-imidazolidine)-2',5'-dione (US 4,436,745, US 4,438,272);
14. 2,7-di-fluoro-5-methoxy-spiro(9H-fluorene-9,4'-imidazolidine)-2',5'-dione (US 4,436,745, US 4,438,272);
15. 7-fluoro-spiro(5H-indenol[1,2-b]pyridine-5,3'-pyrrolidine)-2,5'-dione (US 4,436,745, US 4,438,272);
16. d-cis-6'-chloro-2',3'-dihydro-2'-methyl-spiro-(imidazolidine-4,4'-4'H-pyrano(2,3-b)pyridine)-2,5-dione (US 4,980,357);
17. spiro[imidazolidine-4,5'(6H)-quinoline]-2,5-dione-3'-chloro-7,'8'-dihydro-7'-methyl-(5'-cis) (US 5,066,659);
18. (2S,4S)-6-fluoro-2',5'-dioxospiro(chroman-4,4'-imidazolidine)-2-carboxamide (fidarestat, US 5,447,946); and
19. 2-[(4-bromo-2-fluorophenyl)methyl]-6-fluorospiro[isoquinoline-4(1H),3'-pyrrolidine]-1,2',3,5'(2H)-tetrone (minalrestat, US 5,037,831).

Other aldose reductase inhibitors include compounds of formula A, 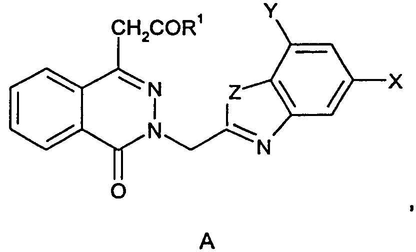 and pharmaceutically acceptable salts thereof, wherein
Z in the compound of formula A is O or S;
R¹ in the compound of formula A is hydroxy or a group capable of being removed *in vivo* to produce a compound of formula A wherein R¹ is OH; and
X and Y in the compound of formula A are the same or different and are selected from hydrogen, trifluoromethyl, fluoro, and chloro.

A preferred subgroup within the above group of aldose reductase inhibitors includes numbered compounds 1, 2, 3, 4, 5, 6, 9, 10, and 17, and the following compounds of formula A:
20. 3,4-dihydro-3-(5-fluorobenzothiazol-2-ylmethyl)-4-oxophthalazin-1-yl-acetic acid [R¹=hydroxy; X=F; Y=H];
21. 3-(5,7-difluorobenzothiazol-2-ylmethyl)-3,4-dihydro-4-oxophthalazin-1-ylacetic acid [R¹=hydroxy; X=Y=F];
22. 3-(5-chlorobenzothiazol-2-ylmethyl)-3,4-dihydro-4-oxophthalazin-1-ylacetic acid [R¹=hydroxy; X=CI; Y=H];
23. 3-(5,7-dichlorobenzothiazol-2-ylmethyl)-3,4-dihydro-4-oxophthalazin-1-ylacetic acid [R¹=hydroxy; X=Y=CI];
24. 3,4-dihydro-4-oxo-3-(5-trifluoromethylbenzoxazol-2-ylmethyl)phthalazin-1-ylacetic acid [R¹=hydroxy; X=CF₃; Y=H];
25. 3,4-dihydro-3-(5-fluorobenzoxazol-2-ylmethyl)-4-oxophthalazin-1-yl-acetic acid [R¹=hydroxy; X=F; Y=H];
26. 3-(5,7-difluorobenzoxazol-2-ylmethyl)-3,4-dihydro-4-oxophthalazin-1-ylacetic acid [R¹=hydroxy; X=Y=F];
27. 3-(5-chlorobenzoxazol-2-ylmethyl)-3,4-dihydro-4-oxophthalazin-1-ylacetic acid [R¹=hydroxy; X=CI; Y=H];
28. 3-(5,7-dichlorobenzoxazol-2-ylmethyl)-3,4-dihydro-4-oxophthalazin-1-ylacetic acid [R¹=hydroxy; X=Y=CI]; and
29. zopolrestat; 1-phthalazineacetic acid, 3,4-dihydro-4-oxo-3-[[5-(trifluoromethyl)-2-benzothiazolyl]methyl]- [R¹=hydroxy; X=trifluoromethyl; Y=H].

In compounds 20-23 and 29, Z is S. In compounds 24-28, Z is O.

Of the above subgroup, compounds 20-29 are more preferred with compound 29 especially preferred.

Said compounds of formula A are prepared as disclosed in US 4,939,140.

The aldose reductase inhibitor compounds of this invention are readily available or can be easily synthesized by those skilled in the art using conventional methods of organic synthesis, particularly in view of the pertinent patent specifications.

The antihypertensive agents which may be used in accordance with this invention are members of different classes of antihypertensive agents, including calcium channel blockers, A-II antagonists, diuretics, beta-adrenergic receptor blockers, vasodilators and alpha-adrenergic receptor blockers.

Calcium channel blockers which are within the scope of this invention include, but are not limited to: bepridil, which may be prepared as disclosed in U.S. Patent No. 3,962,238 or U.S. Reissue No. 30,577; clentiazem, which may be prepared as disclosed in U.S. Patent No. 4,567,175; diltiazem, which may be prepared as disclosed in U.S. Patent No. 3,562,257 fendiline, which may be prepared as disclosed in U.S. Patent No. 3,262,977; gallopamil, which may be prepared as disclosed in U.S. Patent No. 3,261,859; mibefradil, which may be prepared as disclosed in U.S. Patent No. 4,808,605; prenylamine, which may be prepared as disclosed in U.S. Patent No. 3,152,173; semotiadil, which may be prepared as disclosed in U.S. Patent No. 4,786,635; terodiline, which may be prepared as disclosed in U.S. Patent No. 3,371,014; verapamil, which may be prepared as disclosed in U.S. Patent No. 3,261,859; amlodipine, which may be prepared as disclosed in U.S. Patent No. 4,5723,909; aranipine, which may be prepared as disclosed in U.S. Patent No. 4,572,909; barnidipine, which may be prepared as disclosed in U.S. Patent No. 4,220,649; benidipine, which may be prepared as disclosed in European Patent Application Publication No. 106,275; cilnidipine, which may be prepared as disclosed in U.S. Patent No. 4,672,068; efonidipine, which may be prepared as disclosed in U.S. Patent No.4,885,284; elgodipine, which may be prepared as disclosed in U.S. Patent No. 4,952,592; felodipine, which may be prepared as disclosed in U.S. Patent No. 4,264,611; isradipine, which may be prepared as disclosed in U.S. Patent No. 4,466,972; lacidipine, which may be prepared as disclosed in U.S. Patent No. 4,801,599; lercanidipine, which may be prepared as disclosed in U.S. Patent No. 4,705,797; manidipine, which may be prepared as disclosed in U.S. Patent No. 4,892,875; nicardipine, which may be prepared as disclosed in U.S. Patent No. 3,985,758; nifedipine, which may be prepared as disclosed in U.S. Patent No. 3,485,847; nilvadipine, which may be prepared as disclosed in U.S. Patent No. 4,338,322; nimodipine, which may be prepared as disclosed in U.S. Patent No. 3,799,934; nisoldipine, which may be prepared as disclosed in U.S. Patent No. 4,154,839; nitrendipine, which may be prepared as disclosed in U.S. Patent No. 3,799,934; cinnarizine, which may be prepared as disclosed in U.S. Patent No. 2,882,271; flunarizine, which may be prepared as disclosed in U.S. Patent No. 3,773,939; lidoflazine, which may be prepared as disclosed in U.S. Patent No. 3,267,104; lomerizine, which may be prepared as disclosed in U.S. Patent No. 4,663,325; bencyclane, which may be prepared as disclosed in Hungarian Patent No. 151,865; etafenone, which may be prepared as disclosed in German Patent No. 1,265,758; and perhexiline, which may be prepared as disclosed in British Patent No. 1,025,578. Amlodipine besylate, a preferred salt of amlodipine, is disclosed in U.S. Patent No. 4,879,303. The disclosures thereof are incorporated herein by reference.

Angiotensin-II receptor antagonists (A-II antagonists) which are within the scope of this invention include, but are not limited to: candesartan, which may be prepared as disclosed in U.S. Patent No. 5,196,444; eprosartan, which may be prepared as disclosed in U.S. Patent No. 5,185,351; irbesartan, which may be prepared as disclosed in U.S. Patent No. 5,270,317; losartan, which may be prepared as disclosed in U.S. Patent No. 5,138,069; and valsartan, which may be prepared as disclosed in U.S. Patent No. 5,399,578. The disclosures thereof are incorporated herein by reference.

Beta-adrenergic receptor blockers (beta- or β-blockers) which are within the scope of this invention include, but are not limited to: acebutolol, which may be prepared as disclosed in U.S. Patent No. 3,857,952; alprenolol, which may be prepared as disclosed in Netherlands Patent Application No. 6,605,692; amosulalol, which may be prepared as disclosed in U.S. Patent No. 4,217,305; arotinolol, which may be prepared as disclosed in U.S. Patent No. 3,932,400; atenolol, which may be prepared as disclosed in U.S. Patent No. 3,663,607 or 3,836,671; befunolol, which may be prepared as disclosed in U.S. Patent No. 3,853,923; betaxolol, which may be prepared as disclosed in U.S. Patent No. 4,252,984; bevantolol, which may be prepared as disclosed in U.S. Patent No. 3,857,981; bisoprolol, which may be prepared as disclosed in U.S. Patent No. 4,171,370; bopindolol, which may be prepared as disclosed in U.S. Patent No. 4,340,541; bucumolol, which may be prepared as disclosed in U.S. Patent No. 3,663,570; bufetolol, which may be prepared as disclosed in U.S. Patent No. 3,723,476; bufuralol, which may be prepared as disclosed in U.S. Patent No. 3,929,836; bunitrolol, which may be prepared as disclosed in U.S. Patent Nos. 3,940,489 and 3,961,071; buprandolol, which may be prepared as disclosed in U.S. Patent No. 3,309,406; butiridine hydrochloride, which may be prepared as disclosed in French Patent No. 1,390,056; butofilolol, which may be prepared as disclosed in U.S. Patent No. 4,252,825; carazolol, which may be prepared as disclosed in German Patent No. 2,240,599; carteolol, which may be prepared as disclosed in U.S. Patent No. 3,910,924; carvedilol, which may be prepared as disclosed in U.S. Patent No. 4,503,067; celiprolol, which may be prepared as disclosed in U.S. Patent No. 4,034,009; cetamolol, which may be prepared as disclosed in U.S. Patent No. 4,059,622; cloranolol, which may be prepared as disclosed in German Patent No. 2,213,044; dilevalol, which may be prepared as disclosed in Clifton et al., Journal of Medicinal Chemistry, 1982, 25, 670; epanolol, which may be prepared as disclosed in European Patent Publication Application No. 41,491; indenolol, which may be prepared as disclosed in U.S. Patent No. 4,045,482; labetalol, which may be prepared as disclosed in U.S. Patent No. 4,012,444; levobunolol, which may be prepared as disclosed in U.S. Patent No. 4,463,176; mepindolol, which may be prepared as disclosed in Seeman et al., Helv. Chim. Acta, 1971, 54, 241; metipranolol, which may be prepared as disclosed in Czechoslovakian Patent Application No. 128,471; metoprolol, which may be prepared as disclosed in U.S. Patent No. 3,873,600; moprolol, which may be prepared as disclosed in U.S. Patent No. 3,501,769; nadolol, which may be prepared as disclosed in U.S. Patent No. 3,935, 267; nadoxolol, which may be prepared as disclosed in U.S. Patent No. 3,819,702; nebivalol, which may be prepared as disclosed in U.S. Patent No. 4,654,362; nipradilol, which may be prepared as disclosed in U.S. Patent No. 4,394,382; oxprenolol, which may be prepared as disclosed in British Patent No. 1,077,603; perbutolol, which may be prepared as disclosed in U.S. Patent No. 3,551,493; pindolol, which may be prepared as disclosed in Swiss Patent Nos. 469,002 and 472,404; practolol, which may be prepared as disclosed in U.S. Patent No. 3,408,387; pronethalol, which may be prepared as disclosed in British Patent No. 909,357; propranolol, which may be prepared as disclosed in U.S. Patent Nos. 3,337,628 and 3,520,919; sotalol, which may be prepared as disclosed in Uloth et al., Journal of Medicinal Chemistry, 1966, 9, 88; sufinalol, which may be prepared as disclosed in German Patent No. 2,728,641; talindol, which may be prepared as disclosed in U.S. Patent Nos. 3,935,259 and 4,038,313; tertatolol, which may be prepared as disclosed in U.S. Patent No. 3,960,891; tilisolol, which may be prepared as disclosed in U.S. Patent No. 4,129,565; timolol, which may be prepared as disclosed in U.S. Patent No. 3,655,663; toliprolol, which may be prepared as disclosed in U.S. Patent No. 3,432,545; and xibenolol, which may be prepared as disclosed in U.S. Patent No. 4,018,824. The disclosures thereof are incorporated herein by reference.

Endopeptidase inhibitors which are within the scope of this invention include, but are not limited to sampatrilat, which may be prepared as disclosed in European Patent Application Publication No. EP 358398; candoxatril and candoxatrilat, each of which may be prepared as disclosed in European Patent Application Publication No. EP 274234; and omapatrilat, which may be prepared as disclosed in U.S. Patent No. 5,508,272. The disclosures thereof are incorporated herein by reference.

Alpha-adrenergic receptor blockers (alpha- or α-blockers) which are within the scope of this invention include, but are not limited to: amosulalol, which may be prepared as disclosed in U.S. Patent No. 4,217,307; arotinolol, which may be prepared as disclosed in U.S. Patent No. 3,932,400; dapiprazole, which may be prepared as disclosed in U.S. Patent No. 4,252,721; doxazosin, which may be prepared as disclosed in U.S. Patent No. 4,188,390; fenspiride, which may be prepared as disclosed in U.S. Patent No. 3,399,192; indoramin, which may be prepared as disclosed in U.S. Patent No. 3,527,761; labetolol, which may be prepared as disclosed above; naftopidil, which may be prepared as disclosed in U.S. Patent No. 3,997,666; nicergoline, which may be prepared as disclosed in U.S. Patent No. 3,228,943; prazosin, which may be prepared as disclosed in U.S. Patent No. 3,511,836; tamsulosin, which may be prepared as disclosed in U.S. Patent No. 4,703,063; tolazoline, which may be prepared as disclosed in U.S. Patent No. 2,161,938; trimazosin, which may be prepared as disclosed in U.S. Patent No. 3,669,968; and yohimbine, which may be isolated from natural sources according to methods well known to those skilled in the art. The disclosures thereof are incorporated herein by reference.

The term "vasodilator," where used herein, is meant to include cerebral vasodilators, coronary vasodilators and peripheral vasodilators. Cerebral vasodilators within the scope of this invention include, but are not limited to: bencyclane, which may be prepared as disclosed above; cinnarizine, which may be prepared as disclosed above; citicoline, which may be isolated from natural sources as disclosed in Kennedy et al., Journal of the American Chemical Society, 1955, 77, 250 or synthesized as disclosed in Kennedy, Journal of Biological Chemistry, 1956, 222, 185; cyclandelate, which may be prepared as disclosed in U.S. Patent No. 3,663,597; ciclonicate, which may be prepared as disclosed in German Patent No. 1,910,481; diisopropylamine dichloroacetate, which may be prepared as disclosed in British Patent No. 862,248; eburnamonine, which may be prepared as disclosed in Hermann et al., Journal of the American Chemical Society, 1979, 101,1540; fasudil, which may be prepared as disclosed in U.S. Patent No. 4,678,783; fenoxedil, which may be prepared as disclosed in U.S. Patent No. 3,818,021; flunarizine, which may be prepared as disclosed in U.S. Patent No. 3,773,939; ibudilast, which may be prepared as disclosed in U.S. Patent No. 3,850,941; ifenprodil, which may be prepared as disclosed in U.S. Patent No. 3,509,164; lomerizine, which may be prepared as disclosed in U.S. Patent No. 4,663,325; nafronyl, which may be prepared as disclosed in U.S. Patent No. 3,334,096; nicametate, which may be prepared as disclosed in Blicke et al., Journal of the American Chemical Society, 1942, 64, 1722; nicergoline, which may be prepared as disclosed above; nimodipine, which may be prepared as disclosed in U.S. Patent No. 3,799,934; papaverine, which may be prepared as reviewed in Goldberg, Chem. Prod. Chem. News, 1954, 17, 371; pentifylline, which may be prepared as disclosed in German Patent No. 860,217; tinofedrine, which may be prepared as disclosed in U.S. Patent No. 3,563,997; vincamine, which may be prepared as disclosed in U.S. Patent No. 3,770,724; vinpocetine, which may be prepared as disclosed in U.S. Patent No. 4,035,750; and viquidil, which may be prepared as disclosed in U.S. Patent No. 2,500,444. The disclosures thereof are incorporated herein by reference.

Coronary vasodilators within the scope of this invention include, but are not limited to: amotriphene, which may be prepared as disclosed in U.S. Patent No. 3,010,965; bendazol, which may be prepared as disclosed in J. Chem. Soc. 1958, 2426; benfurodil hemisuccinate, which may be prepared as disclosed in U.S. Patent No. 3,355,463; benziodarone, which may be prepared as disclosed in U.S. Patent No. 3,012,042; chloracizine, which may be prepared as disclosed in British Patent No. 740,932; chromonar, which may be prepared as disclosed in U.S. Patent No. 3,282,938; clobenfural, which may be prepared as disclosed in British Patent No. 1,160,925; clonitrate, which may be prepared from propanediol according to methods well known to those skilled in the art, e.g., see *Annalen,* 1870, 155, 165; cloricromen, which may be prepared as disclosed in U.S. Patent No. 4,452,811; dilazep, which may be prepared as disclosed in U.S. Patent No. 3,532,685; dipyridamole, which may be prepared as disclosed in British Patent No. 807,826; droprenilamine, which may be prepared as disclosed in German Patent No. 2,521,113; efloxate, which may be prepared as disclosed in British Patent Nos. 803,372 and 824,547; erythrityl tetranitrate, which may be prepared by nitration of erythritol according to methods well-known to those skilled in the art; etafenone, which may be prepared as disclosed in German Patent No. 1,265,758; fendiline, which may be prepared as disclosed in U.S. Patent No. 3,262,977; floredil, which may be prepared as disclosed in German Patent No. 2,020,464; ganglefene, which may be prepared as disclosed in U.S.S.R. Patent No. 115,905; hexestrol, which may be prepared as disclosed in U.S. Patent No. 2,357,985; hexobendine, which may be prepared as disclosed in U.S. Patent No. 3,267,103; itramin tosylate, which may be prepared as disclosed in Swedish Patent No. 168,308; khellin, which may be prepared as disclosed in Baxter et al., Journal of the Chemical Society, 1949, S 30; lidoflazine, which may be prepared as disclosed in U.S. Patent No. 3,267,104; mannitol hexanitrate, which may be prepared by the nitration of mannitol according to methods well-known to those skilled in the art; medibazine, which may be prepared as disclosed in U.S. Patent No. 3,119,826; nitroglycerin; pentaerythritol tetranitrate, which may be prepared by the nitration of pentaerythritol according to methods well-known to those skilled in the art; pentrinitrol, which may be prepared as disclosed in German Patent No. 638,422-3; perhexilline, which may be prepared as disclosed above; pimefylline, which may be prepared as disclosed in U.S. Patent No. 3,350,400; prenylamine, which may be prepared as disclosed in U.S. Patent No. 3,152,173; propatyl nitrate, which may be prepared as disclosed in French Patent No. 1,103,113; trapidil, which may be prepared as disclosed in East German Patent No. 55,956; tricromyl, which may be prepared as disclosed in U.S. Patent No. 2,769,015; trimetazidine, which may be prepared as disclosed in U.S. Patent No. 3,262,852; trolnitrate phosphate, which may be prepared by nitration of triethanolamine followed by precipitation with phosphoric acid according to methods well-known to those skilled in the art; visnadine, which may be prepared as disclosed in U.S. Patent Nos. 2,816,118 and 2,980,699. The disclosures thereof are incorporated herein by reference.

Peripheral vasodilators within the scope of this invention include, but are not limited to: aluminum nicotinate, which may be prepared as disclosed in U.S. Patent No. 2,970,082; bamethan, which may be prepared as disclosed in Corrigan et al., Journal of the American Chemical Society, 1945, 67, 1894; bencyclane, which may be prepared as disclosed above; betahistine, which may be prepared as disclosed in Walter et al.; Journal of the American Chemical Society, 1941, 63, 2771; bradykinin, which may be prepared as disclosed in Hamburg et al., Arch. Biochem. Biophys., 1958, 76, 252; brovincamine, which may be prepared as disclosed in U.S. Patent No. 4,146,643; bufeniode, which may be prepared as disclosed in U.S. Patent No. 3,542,870; buflomedil, which may be prepared as disclosed in U.S. Patent No. 3,895,030; butalamine, which may be prepared as disclosed in U.S. Patent No. 3,338,899; cetiedil, which may be prepared as disclosed in French Patent Nos. 1,460,571; ciclonicate, which may be prepared as disclosed in German Patent No. 1,910,481; cinepazide, which may be prepared as disclosed in Belgian Patent No. 730,345; cinnarizine, which may be prepared as disclosed above; cyclandelate, which may be prepared as disclosed above; diisopropylamine dichloroacetate, which may be prepared as disclosed above; eledoisin, which may be prepared as disclosed in British Patent No. 984,810; fenoxedil, which may be prepared as disclosed above; flunarizine, which may be prepared as disclosed above; hepronicate, which may be prepared as disclosed in U.S. Patent No. 3,384,642; ifenprodil, which may be prepared as disclosed above; iloprost, which may be prepared as disclosed in U.S. Patent No. 4,692,464; inositol niacinate, which may be prepared as disclosed in Badgett et al., Journal of the American Chemical Society, 1947, 69, 2907; isoxsuprine, which may be prepared as disdosed in U.S. Patent No. 3,056,836; kallidin, which may be prepared as disclosed in *Biochem. Biophys. Res. Commun.,* **1961, 6,** 210; kallikrein, which may be prepared as disclosed in German Patent No. 1,102,973; moxisylyte, which may be prepared as disclosed in German Patent No. 905,738; nafronyl, which may be prepared as disclosed above; nicametate, which may be prepared as disclosed above; nicergoline, which may be prepared as disclosed above; nicofuranose, which may be prepared as disclosed in Swiss Patent No. 366,523; nylidrin, which may be prepared as disclosed in U.S. Patent Nos. 2,661,372 and 2,661,373; pentifylline, which may be prepared as disclosed above; pentoxifylline, which may be prepared as disclosed in U.S. Patent No. 3,422,107; piribedil, which may be prepared as disclosed in U.S. Patent No. 3,299,067; prostaglandin E₁, which may be prepared by any of the methods referenced in the Merck Index, Twelfth Edition, Budaveri, Ed., New Jersey, 1996, p. 1353; suloctidil, which may be prepared as disclosed in German Patent No. 2,334,404; tolazoline, which may be prepared as disclosed in U.S. Patent No. 2,161,938; and xanthinol niacinate, which may be prepared as disclosed in German Patent No. 1,102,750 or Korbonits et al., Acta. Pharm. Hung., 1968, 38, 98. The disclosures thereof are incorporated herein by reference.

The term "diuretic," within the scope of this invention, is meant to include diuretic benzothiadiazine derivatives, diuretic organomercurials, diuretic purines, diuretic steroids, diuretic sulfonamide derivatives, diuretic uracils and other diuretics such as amanozine, which may be prepared as disclosed in Austrian Patent No. 168,063; amiloride, which may be prepared as disclosed in Belgian Patent No. 639,386; arbutin, which may be prepared as disclosed in Tschitschibabin, Annalen, 1930, 479, 303; chlorazanil, which may be prepared as disclosed in Austrian Patent No. 168,063; ethacrynic acid, which may be prepared as disdosed in U.S. Patent No. 3,255,241; etozolin, which may be prepared as disclosed in U.S. Patent No. 3,072,653; hydracarbazine, which may be prepared as disclosed in British Patent No. 856,409; isosorbide, which may be prepared as disclosed in U.S. Patent No. 3,160,641; mannitol; metochalcone, which may be prepared as disclosed in Freudenberg et al., Ber., 1957, 90, 957; muzolimine, which may be prepared as disclosed in U.S. Patent No. 4,018,890; perhexiline, which may be prepared as disclosed above; ticrynafen, which may be prepared as disclosed in U.S. Patent No. 3,758,506; triamterene which may be prepared as disclosed in U.S. Patent No. 3,081,230; and urea. The disclosures thereof are incorporated herein by reference.

Diuretic benzothiadiazine derivatives within the scope of this invention include, but are not limited to: althiazide, which may be prepared as disclosed in British Patent No. 902,658; bendroflumethiazide, which may be prepared as disclosed in U.S. Patent No. 3,265,573; benzthiazide, McManus et al., 136th Am. Soc. Meeting (Atlantic City, September 1959), Abstract of papers, pp 13-O; benzylhydrochlorothiazide, which may be prepared as disclosed in U.S. Patent No. 3,108,097; buthiazide, which may be prepared as disclosed in British Patent Nos. 861,367 and 885,078; chlorothiazide, which may be prepared as disclosed in U.S. Patent Nos. 2,809,194 and 2,937,169; chlorthalidone, which may be prepared as disclosed in U.S. Patent No. 3,055,904; cyclopenthiazide, which may be prepared as disclosed in Belgian Patent No. 587,225; cyclothiazide, which may be prepared as disclosed in Whitehead et al., Journal of Organic Chemistry, 1961, 26, 2814; epithiazide, which may be prepared as disclosed in U.S. Patent No. 3,009,911; ethiazide, which may be prepared as disclosed in British Patent No. 861,367; fenquizone, which may be prepared as disclosed in U.S. Patent No. 3,870,720; indapamide, which may be prepared as disclosed in U.S. Patent No. 3,565,911; hydrochlorothiazide, which may be prepared as disclosed in U.S. Patent No. 3,164,588; hydroflumethiazide, which may be prepared as disclosed in U.S. Patent No. 3,254,076; methyclothiazide, which may be prepared as disclosed in Close et al., Journal of the American Chemical Society, 1960, 82, 1132; meticrane, which may be prepared as disclosed in French Patent Nos. M2790 and 1,365,504; metolazone, which may be prepared as disclosed in U.S. Patent No. 3,360,518; paraflutizide, which may be prepared as disclosed in Belgian Patent No. 620,829; polythiazide, which may be prepared as disclosed in U.S. Patent No. 3,009,911; quinethazone, which may be prepared as disclosed in U.S. Patent No. 2,976,289; teclothiazide, which may be prepared as disclosed in Close et al., Journal of the American Chemical Society, 1960, 82, 1132; and trichlormethiazide, which may be prepared as dislcosed in deStevens et al., Experientia, 1960, 16, 113. The disclosures thereof are incorporated herein by reference.

Diuretic sulfonamide derivatives within the scope of this invention include, but are not limited to: acetazolamide, which may be prepared as disclosed in U.S. Patent No. 2,980,679; ambuside, which may be prepared as disclosed in U.S. Patent No. 3,188,329; azosemide, which may be prepared as disclosed in U.S. Patent No. 3,665,002; bumetanide, which may be prepared as disclosed in U.S. Patent No. 3,634,583; butazolamide, which may be prepared as disclosed in British Patent No. 769,757; chloraminophenamide, which may be prepared as disclosed in U.S. Patent Nos. 2,809,194, 2,965,655 and 2,965,656; clofenamide, which may be prepared as disclosed in Olivier, Rec. Trav. Chim., 1918, 37, 307; clopamide, which may be prepared as disclosed in U.S. Patent No. 3,459,756; clorexolone, which may be prepared as disclosed in U.S. Patent No. 3,183,243; disulfamide, which may be prepared as disclosed in British Patent No. 851,287; ethoxolamide, which may be prepared as disclosed in British Patent No. 795,174; furosemide, which may be prepared as disclosed in U.S. Patent No. 3,058,882; mefruside, which may be prepared as disclosed in U.S. Patent No. 3,356,692; methazolamide, which may be prepared as disclosed in U.S. Patent No. 2,783,241; piretanide, which may be prepared as disclosed in U.S. Patent No. 4,010,273; torasemide, which may be prepared as disclosed in U.S. Patent No. 4,018,929; tripamide, which may be prepared as disclosed in Japanese Patent No. 73 05,585; and xipamide, which may be prepared as disclosed in U.S. Patent No. 3,567,777. The disclosures thereof are incorporated herein by reference.

The expression "pharmaceutically acceptable salts" includes both pharmaceutically acceptable acid addition salts and pharmaceutically acceptable cationic salts, where appropriate. The expression "pharmaceutically-acceptable cationic salts" is intended to define but is not limited to such salts as the alkali metal salts, (e.g., sodium and potassium), alkaline earth metal salts (e.g., calcium and magnesium), aluminum salts, ammonium salts, and salts with organic amines such as benzathine (N,N'-dibenzylethylenediamine), choline, diethanolamine, ethylenediamine, meglumine (N-methylglucamine), benethamine (N-benzylphenethylamine), diethylamine, piperazine, tromethamine (2-amino-2-hydroxymethyl-1,3-propanediol) and procaine. The expression "pharmaceutically-acceptable acid addition salts" is intended to define but is not limited to such salts as the hydrochloride, hydrobromide, sulfate, hydrogen sulfate, phosphate, hydrogen phosphate, dihydrogenphosphate, acetate, succinate, citrate, methanesulfonate (mesylate) and p-toluenesulfonate (tosylate) salts.

Pharmaceutically acceptable salts of the aldose reductase inhibitors of this invention may be readily prepared by reacting the free acid form of said aldose reductase inhibitor with an appropriate base, usually one equivalent, in a co-solvent. Typical bases are sodium hydroxide, sodium methoxide, sodium ethoxide, sodium hydride, potassium methoxide, magnesium hydroxide, calcium hydroxide, benzathine, choline, diethanolamine, piperazine and tromethamine. The salt is isolated by concentration to dryness or by addition of a non-solvent. In many cases, salts are preferably prepared by mixing a solution of the acid with a solution of a different salt of the cation (sodium or potassium ethylhexanoate, magnesium oleate), and employing a solvent (e.g., ethyl acetate) from which the desired cationic salt precipitates, or can be otherwise isolated by concentration and/or addition of a non-solvent.

The acid addition salts of the aldose reductase inhibitors of this invention may be readily prepared by reacting the free base form of said aldose reductase inhibitor with the appropriate acid. When the salt is of a monobasic acid (e.g., the hydrochloride, the hydrobromide, the p-toluenesulfonate, the acetate), the hydrogen form of a dibasic acid (e.g., the hydrogen sulfate, the succinate) or the dihydrogen form of a tribasic acid (e.g., the dihydrogen phosphate, the citrate), at least one molar equivalent and usually a molar excess of the acid is employed. However when such salts as the sulfate, the hemisuccinate, the hydrogen phosphate or the phosphate are desired, the appropriate and exact chemical equivalents of acid will generally be used. The free base and the acid are usually combined in a co-solvent from which the desired salt precipitates, or can be otherwise isolated by concentration and/or addition of a non-solvent.

The pharmaceutically acceptable acid addition and cationic salts of the antihypertensive agents used in the combination of this invention may be prepared in a manner analogous to that described for the preparation of the pharmaceutically acceptable acid addition and cationic salts of said aldose reductase inhibitors, but substituting the desired antihypertensive agent for said aldose reductase inhibitor.

In addition, the aldose reductase inhibitors and antihypertensive agents which may be used in accordance with this invention, prodrugs thereof and pharmaceutically acceptable salts thereof, may occur as hydrates or solvates. Said hydrates and solvates are also within the scope of the invention.

This invention relates both to methods of treating diabetic complications in which the ARI and antihypertensive agent are administered together, as part of the same pharmaceutical composition, and to methods in which these two active agents are administered separately, as part of an appropriate dosage regimen designed to obtain the benefits of the combination therapy. The appropriate dosage regimen, the amount of each dose administered and the intervals between doses of the active agents will depend upon the ARI and the antihypertensive agent being used, the type of pharmaceutical formulations being used, the characteristics of the subject being treated and the severity of the complications. Generally, in carrying out the methods of this invention, an effective dosage for the aldose reductase inhibitors of this invention is in the range of about 0.01 mg/kg/day to about 100 mg/kg/day in single or divided doses, preferably 0.1 mg/kg/day to 20 mg/kg/day in single or divided doses and the antihypertensive agent will be administered in single or divided doses. Antihypertensive agents will generally be administered in amounts ranging from about 0.01 mg/kg/day to about 500 mg/kg/day in single or divided doses, preferably 10 mg to about 300 mg per day for an average subject, depending upon the antihypertensive agent and the route of administration. However, some variation in dosage will necessarily occur depending on the condition of the subject being treated. The prescribing physician will, in any event, determine the appropriate dose for the individual subject.

Administration of the pharmaceutical compositions of this invention can be via any method which delivers a composition of this invention preferentially to the desired tissue (e.g., nerve, kidney, retina and/or cardiac tissues). These methods include oral routes, parenteral, intraduodenal routes, etc. Generally, the compositions of the present invention are administered in single (e.g., once daily) or multiple doses or via constant infusion.

Pharmaceutical compositions comprising an aldose reductase inhibitor, a prodrug thereof or a pharmaceutically acceptable salt of said aldose reductase inhibitor or said prodrug and an antihypertensive agent, a prodrug thereof or a pharmaceutically acceptable salt of said antihypertensive agent or said prodrug are hereinafter referred to, collectively, as "the active compositions of this invention."

The active compositions of this invention may be administered to a subject in need of treatment by a variety of conventional routes of administration, including orally, topically, parenterally, e.g., intravenously, subcutaneously or intramedullary. Further, the active compositions of this invention may be administered intranasally, as a rectal suppository or using a "flash" formulation, i.e., allowing the medication to dissolve in the mouth without the need to use water.

The active compositions of this invention may be administered alone or in combination with pharmaceutically acceptable carriers, vehicles or diluents, in either single or multiple doses. Suitable pharmaceutical carriers, vehicles and diluents include inert solid diluents or fillers, sterile aqueous solutions and various organic solvents. The pharmaceutical compositions formed by combining the active compositions of this invention and the pharmaceutically acceptable carriers, vehicles or diluents are then readily administered in a variety of dosage forms such as tablets, powders, lozenges, syrups, injectable solutions and the like. These pharmaceutical compositions can, if desired, contain additional ingredients such as flavorings, binders, excipients and the like. Thus, for purposes of oral administration, tablets containing various excipients such as sodium citrate, calcium carbonate and calcium phosphate may be employed along with various disintegrants such as starch, alginic acid and certain complex silicates, together with binding agents such as polyvinylpyrrolidone, sucrose, gelatin and acacia. Additionally, lubricating agents such as magnesium stearate, sodium lauryl sulfate and talc are often useful for tabletting purposes. Solid compositions of a similar type may also be employed as fillers in soft and hard filled gelatin capsules. Preferred materials for this include lactose or milk sugar and high molecular weight polyethylene glycols. When aqueous suspensions or elixirs are desired for oral administration, the essential active ingredient therein may be combined with various sweetening or flavoring agents, coloring matter or dyes and, if desired, emulsifying or suspending agents, together with diluents such as water, ethanol, propylene glycol, glycerin and combinations thereof.

For parenteral administration, solutions of the active compositions of this invention in sesame or peanut oil, aqueous propylene glycol, or in sterile aqueous solutions may be employed. Such aqueous solutions should be suitably buffered if necessary and the liquid diluent first rendered isotonic with sufficient saline or glucose. These particular aqueous solutions are especially suitable for intravenous, intramuscular, subcutaneous and intraperitoneal administration. In this connection, the sterile aqueous media employed are all readily available by standard techniques known to those skilled in the art.

Generally, a composition of this invention is administered orally, or parenterally (e.g., intravenous, intramuscular, subcutaneous or intramedullary). Topical administration may also be indicated, for example, where the patient is suffering from gastrointestinal disorders or whenever the medication is best applied to the surface of a tissue or organ as determined by the attending physician.

For buccal administration the composition (two active agents administered together or separately) may take the form of tablets or lozenges formulated in a conventional manner.

For intranasal administration or administration by inhalation, the active compounds of the invention (two active agents administered together or separately) are conveniently delivered in the form of a solution or suspension from a pump spray container that is squeezed or pumped by the patient or as an aerosol spray presentation from a pressurized container or a nebulizer, with the use of a suitable propellant, e.g., dichlorodifluoromethane, trichlorofluoromethane, dichlorotetrafluoroethane, carbon dioxide or other suitable gas. In the case of a pressurized aerosol, the dosage unit may be determined by providing a valve to deliver a metered amount. The pressurized container or nebulizer may contain a solution or suspension of the active compound. Capsules and cartridges (made, for example, from gelatin) for use in an inhaler or insufflator may be formulated containing a powder mix of a compound or compounds of the invention and a suitable powder base such as lactose or starch.

For purposes of transdermal (e.g.,topical) administration, dilute sterile, aqueous or partially aqueous solutions (usually in about 0.1% to 5% concentration), otherwise similar to the above parenteral solutions, are prepared.

Methods of preparing various pharmaceutical compositions with a certain amount of active ingredient are known, or will be apparent in light of this disclosure, to those skilled in this art. For examples of methods of preparing pharmaceutical compositions, see Remington's Pharmaceutical Sciences, Mack Publishing Company, Easton, Pa., 19th Edition (1995).

The active compositions of this invention contain an amount of both an aldose reductase inhibitor, a prodrug thereof or a pharmaceutically acceptable salt of said aldose reductase inhibitor and of an antihypertensive agent, a prodrug thereof or a pharmaceutically acceptable salt of said antihypertensive agent or said prodrug. The amount of each of those ingredients may independently be, for example, 0.0001%- 95% of the total amount of the composition, where the total amount may not, of course, exceed 100%. In any event, the composition or formulation to be administered will contain a quantity of each of the components of the composition according to the invention in an amount effective to treat the disease/condition of the subject being treated.

Since the present invention has an aspect that relates to the treatment of the disease/conditions described herein with a combination of active ingredients which may be administered separately, the invention also relates to combining separate pharmaceutical compositions in kit form. The kit comprises two separate pharmaceutical compositions: an aldose reductase inhibitor, a prodrug thereof or a salt of such aldose reductase inhibitor or prodrug; and an antihypertensive agent, a prodrug thereof or a salt of said antihypertensive agent or prodrug as described above. The kit comprises a container for containing the separate compositions such as a divided bottle or a divided foil packet. Typically the kit comprises directions for the administration of the separate components. The kit form is particularly advantageous when the separate components are preferably administered in different dosage forms (e.g., oral and parenteral), are administered at different dosage intervals, or when titration of the individual components of the combination is desired by the prescribing physician.

An example of such a kit is a so-called blister pack. Blister packs are well known in the packaging industry and are being widely used for the packaging of pharmaceutical unit dosage forms (tablets, capsules, and the like). Blister packs generally consist of a sheet of relatively stiff material covered with a foil of a preferably transparent plastic material. During the packaging process recesses are formed in the plastic foil. The recesses have the size and shape of the tablets or capsules to be packed. Next, the tablets or capsules are placed in the recesses and the sheet of relatively stiff material is sealed against the plastic foil at the face of the foil which is opposite from the direction in which the recesses were formed. As a result, the tablets or capsules are sealed in the recesses between the plastic foil and the sheet. Preferably the strength of the sheet is such that the tablets or capsules can be removed from the blister pack by manually applying pressure on the recesses whereby an opening is formed in the sheet at the place of the recess. The tablet or capsule can then be removed via said opening.

It may be desirable to provide a memory aid on the kit, e.g., in the form of numbers next to the tablets or capsules whereby the numbers correspond with the days of the regimen which the tablets or capsules so specified should be ingested. Another example of such a memory aid is a calendar printed on the card, e.g., as follows "First Week, Monday, Tuesday, ...etc.... Second Week, Monday, Tuesday,..." etc. Other variations of memory aids will be readily apparent. A "daily dose" can be a single tablet or capsule or several pills or capsules to be taken on a given day. Also, a daily dose of the aldose reductase inhibitor can consist of one tablet or capsule while a daily dose of the antihypertensive agent can consist of several tablets or capsules and vice versa. The memory aid should reflect this.

In another specific embodiment of the invention, a dispenser designed to dispense the daily doses one at a time in the order of their intended use is provided. Preferably, the dispenser is equipped with a memory-aid, so as to further facilitate compliance with the regimen. An example of such a memory-aid is a mechanical counter which indicates the number of daily doses that has been dispensed. Another example of such a memory-aid is a battery-powered micro-chip memory coupled with a liquid crystal readout, or audible reminder signal which, for example, reads out the date that the last daily dose has been taken and/or reminds one when the next dose is to be taken.

The compositions of this invention generally will be administered in a convenient formulation.

## Claims

1. A pharmaceutical composition comprising an aldose reductase inhibitor (ARI), a prodrug thereof or a pharmaceutically acceptable salt of said ARI or said prodrug; an antihypertensive agent, a prodrug thereof or a pharmaceutically acceptable salt of said antihypertensive agent or said prodrug; and a pharmaceutically acceptable carrier, vehicle or diluent, provided that said antihypertensive agent is not an ACE inhibitor.

2. A composition of claim 1 wherein said ARI is fidarestat, epalrestat, minalrestat, SPR-210, zenarastat or zopolrestat, a prodrug thereof or a pharmaceutically acceptable salt of said ARI or of said prodrug.

3. A composition of claim 2 wherein said ARI is zopolrestat, a prodrug thereof or a pharmaceutically acceptable salt of zopolrestat or of said prodrug.

4. A composition of claim 1 wherein said antihypertensive agent is a calcium channel blocker, an A-II antagonist, a diuretic, a neutral endopeptidase inhibitor, a beta-adrenergic receptor blocker or an alpha-adrenergic receptor blocker, a prodrug of said antihypertensive agent or a pharmaceutically acceptable salt of said antihypertensive agent or of said prodrug.

5. A composition of claim 4 wherein said antihypertensive agent is a calcium channel blocker, said calcium channel blocker being verapamil, diltiazem, mibefradil, isradipine, lacidipine, nicardipine, nifedipine, amlodipine, nimodipine, nisoldipine, nitrendipine or felodipine, a prodrug of said calcium channel blocker or a pharmaceutically acceptable salt of said calcium channel blocker or of said prodrug.

6. A composition of claim 5 wherein said calcium channel blocker is felodipine, amlodipine, nifedipine, a prodrug of said calcium channel blocker or a pharmaceutically acceptable salt of said calcium channel blocker or of said prodrug.

7. A composition of claim 6 wherein said ARI is defined in claims 2 or 3.

8. A composition of claim 4 wherein said antihypertensive agent is an A-II antagonist, said A-II antagonist being losartan, irbesartan or valsartan, a prodrug of said A-II antagonist or a pharmaceutically acceptable salt of said A-II antagonist or of said prodrug.

9. A compositon of claim 8 wherein said ARI is defined in claims 2 or 3.

10. A pharmaceutical composition of claim 4 wherein said antihypertensive agent is a diuretic, said diuretic being amiloride, bendroflumethiazide, a prodrug of said diuretic or a pharmaceutically acceptable salt of said diuretic or of said prodrug.

11. A composition of claim 10 wherein said ARI is defined in claims 2 or 3.

12. A pharmaceutical composition of claim 4 wherein said antihypertensive agent is a beta-adrenergic receptor blocker, said beta-adrenergic receptor blocker being carvedilol, a prodrug thereof or a pharmaceutically acceptable salt thereof or of said prodrug.

13. A composition of claim 12 wherein said ARI is as defined in claims 2 or 3.

14. A pharmaceutical composition of claim 4 wherein said antihypertensive agent is an alpha-adrenergic receptor blocker, said alpha-adrenergic receptor blocker being doxazosin, prazosin, trimazosin, a prodrug of said alpha-adrenergic receptor blocker or a pharmaceutically acceptable salt of said alpha-adrenergic receptor blocker or of said prodrug.

15. A composition of claim 14 wherein said ARI is as defined in claims 2 or 3.

16. A composition of claim 4 wherein said antihypertensive agent is a neutral endopeptidase inhibitor, said neutral endopeptidase inhibitor being candoxatril, candoxatrilat, samplatrilat or omapatrilat, a prodrug thereof or a pharmaceutically acceptable salt of said neutral endopeptidase inhibitor or said prodrug.

17. A composition of claim 16 wherein said ARI is defined in claims 2 or 3.

18. The use of a composition as defined in any preceding claim for the manufacture of a medicament for treating a diabetic complication in a mammal.

19. The use according to claim 18 wherein said antihypertensive agent is a calcium channel blocker, an A-II antagonist, a diuretic, a beta-adrenergic receptor blcoker or an alpha-adrenergic receptor blocker, a pharmaceutically acceptable salt of any of said agents or a prodrug or any of said agents or of said salts.

20. The use according to claim 19 wherein said ARI is defined in claims 2 or 3.

21. The use according to any one of claims 18 to 20 wherein said diabetic complication is dibaetic neuropathy.

22. The use according to any one of claims 18 to 20 wherein said diabetic complication is diabetic.

23. The use according to any one of claims 18 to 20 wherein said diabetic complication is diabetic cardiomyopathy.

24. The use according to any one of claims 18 to 20 wherein said diabetic complication is diabetic retinopathy.

25. The use according to any one of claims 18 to 20 wherein said diabetic complication is cataracts.

26. The use according to any one of claims 18 to 20 wherein said diabetic complication is myocardial infarction.

27. The use of an ARI, a prodrug thereof or a pharmaceutically acceptable salt of said ARI or of said prodrug and an antihypertensive agent, a prodrug thereof or a pharmaceutically acceptable salt of said antihypertensive agent or of said prodrug, provided that said antihypertensive agent is not an ACE inhibitor for the manufacture of a medicament for treating a diabetic complication in a mammal.

28. The use according to claim 27 wherein said antihypertensive agent is a calcium channel blocker, an A-II antagonist, a diuretic, a neutral endopeptidase inhibitor, a beta-adrenergic receptor blocker or an alpha-adrenergic receptor blocker, a pharmaceutically acceptable salt of any of said agents or a prodrug of any of said agents or of said salts.

29. The use according to claim 28 wherein said ARI is as defined in claims 2 or 3.

30. The use according to claim 28 wherein the ARI, prodrug thereof or pharmaceutically acceptable salt of said ARI or said prodrug and the antihypertensive agent, prodrug thereof or pharmaceutically acceptable salt of said antihypertensive agent are administered separately.

31. The use according to claim 28 wherein the ARI, prodrug thereof or pharmaceutically acceptable salt of said ARI or said prodrug and the antihypertensive agent, prodrug thereof or pharmaceutically acceptable salt of said antihypertensive agent are administered together.

32. A kit comprising:
a) a first dosage form comprising an aldose reductase inhibitor (ARI), a prodrug thereof or a pharmaceutically acceptable salt of said ARI or said prodrug and a pharmaceutically acceptable carrier, vehicle or diluent;
b) a second unit dosage form comprising an antihypertensive agent, a prodrug thereof or a pharmaceutically acceptable salt of said antihypertensive agent or said prodrug and a pharmaceutically acceptable carrier, vehicle or diluent, provided that said antihypertensive agent is not an ACE inhibitor; and
c) a container.

33. A kit of claim 32 wherein said antihypertensive agent is a calcium channel blocker, an A-II antagonist, a diuretic, a neutral endopeptidase inhibitor, a beta-adrenergic receptor blocker or an alpha-adrenergic receptor blocker, a pharmaceutically acceptable salt of any of said agents or a prodrug of any of said agents or of said salts.

34. A kit of claim 33 wherein said ARI is as defined in claims 2 or 3.
